# DEMANDE DE BREVET EUROPEEN

(11) **EP 2 111 840 A1**
(43) Date de publication de la demande: **28.10.2009**
(21) Numéro de dépôt: 09158173.6
(22) Date de dépôt: 17.04.2009
(51) Int. Cl.: A61K 8/04, A61K 8/19, A61K 8/44, A61K 8/85, A61Q 1/14, A61Q 9/02, A61Q 19/10

(54) **Composition à effet chauffant avec tensioactifs moussants**

(30) Priorité: 25.04.2008 FR 0852811
(71) Demandeur: L'Oréal, 75008 Paris (FR)
(72) Inventeur: Senee, Jérôme, 91510 Lardy (FR); Compain, Delphine, 75013 Paris (FR)
(74) Mandataire: Duvert, Sandra

(57) **Abrégé**

L'invention se rapporte à une composition cosmétique comprenant dans un milieu anhydre, au moins un sel métallique exothermique, au moins au moins un premier tensioactif moussant amphotère et un au moins un second tensioactif moussant anionique, ladite composition comprenant moins de 1 % d'agent gélifiant.

## Description

La présente invention a pour objet une composition cosmétique anhydre comprenant un sel métallique, au moins deux tensioactifs moussants, ladite composition comprenant moins de 1 % d'agent gélifiant.

Il est connu d'utiliser des compositions cosmétiques anhydres exothermiques, c'est-à-dire ayant un effet chauffant lors de l'application sur la peau. Les produits chauffants peuvent contenir différentes matières premières qui, en présence d'eau, dégagent de la chaleur. Comme matières premières de ce type, on peut citer par exemple les polyols comme la glycérine et les glycols, les sels comme le chlorure de calcium ou le sulfate de magnésium, ou les zéolithes. Les polyols seuls donnent un effet chaud qui est faible. Les sels ou les zéolithes sont performants seuls ou en association avec les polyols, mais, il est néanmoins nécessaire d'incorporer dans les compositions, un fort pourcentage de sels (par exemple 10 à 30%) pour avoir un effet chaud satisfaisant, c'est-à-dire une augmentation de 10°C à 30°C lors de l'humidification par ajout d'eau ou lors de l'application sur la peau humide. Malheureusement, les sels sont connus pour déstabiliser les compositions cosmétiques, et la présence d'agents gélifiant tels que par exemple des silices hydrophiles ou hydrophobes est alors nécessaires pour stabiliser les compositions.

La demanderesse a trouvé de manière surprenante qu'il était possible de formuler de des compositions cosmétiques à effet chauffant comprenant un sel, stables et qui sont contiennent peu ou pas d'agents gélifiants, et ce grâce à la présence d'au moins deux tensioactifs moussants en un ratio défini.

Ainsi, l'invention se rapporte à une composition cosmétique comprenant dans un milieu anhydre, au moins un sel métallique exothermique, au moins un premier tensioactif moussant amphotère et un au moins un second tensioactif moussant anionique présents en un ratio tensioactif anionique / tensioactif amphotère allant de 0,5 à 2, ladite composition comprenant moins de 1 % d'agent gélifiant.

La composition de l'invention est exothermique, c'est-à-dire qu'elle a un effet chauffant, et donc que l'utilisateur ressent un échauffement lors de l'application de la dite composition sur la peau humide ou humidifiée. C'est une composition dont la température en présence d'eau (l'eau ajoutée lors de l'utilisation ou bien l'eau présente dans la peau) peut s'élever de plusieurs degrés (un à vingt degrés) de manière instantanée. Cet effet chauffant va permettre l'ouverture des pores de la peau et donc un meilleur nettoyage de la peau.

La composition selon l'invention est constituée d'un milieu anhydre. On entend ici par « anhydre » un milieu quasiment anhydre, c'est-à-dire comprenant généralement moins de 6 % en poids d'eau, de préférence moins de 4 % en poids d'eau, et encore mieux moins de 1 % en poids d'eau par rapport au poids total de la composition. Elle est de préférence totalement anhydre et donc exempte d'eau.

La composition de l'invention étant une composition cosmétique et donc destinée à une application topique, elle comporte un milieu physiologiquement acceptable, c'est-à-dire compatible avec la peau, les muqueuses et/ou les fibres kératiniques.

### Tensioactifs moussants

Le premier tensioactif amphotère peut être choisi par exemple parmi les bétaïnes, les N-alkylamidobétaïnes et leurs dérivés, les sultaïnes, les alkyl polyaminocarboxylates, les alkylamphoacétates et leurs mélanges.
1) Comme bétaïnes, on peut citer notamment les alkylbétaïnes comme par exemple la cocobétaïne comme le produit commercialisé sous la dénomination DEHYTON AB-30^{®} par la société Cognis, la laurylbétaïne comme le produit commercialisé sous la dénomination GENAGEN KB^{®} par la société Clariant, la laurylbétaïne oxyethylénée (10 OE), comme le produit commercialisé sous la dénomination LAURYLETHER (10 OE) BETAINE^{®} par la société Shin Nihon Rica, la stéarylbétaïne oxyéthylénée (10 OE) comme le produit commercialisé sous la dénomination STEARYLETHER (10 OE) BETAINE^{®} par la société Shin Nihon Rica.
   Parmi les N-alkylamidobétaines et leurs dérivés, on peut citer par exemple la cocamidopropyl bétaine commercialisée sous la dénomination LEBON 2000 HG^{®} par la société Sanyo, ou commercialisée sous la dénomination EMPIGEN BB^{®} par la société Albright & Wilson, la lauramidopropyl bétaïne commercialisée sous la dénomination REWOTERIC AMB12P^{®} par la société Witco.
2) Comme sultaines, on peut citer les hydroxylsultaïnes, telles que la cocamidopropyl hydroxysultaïne comme le produit commercialisé sous la dénomination REWOTERIC AM CAS par la société Golschmidt-Degussa, ou le produit commercialisé sous la dénomination CROSULTAINE C-50^{®} par la société Croda.
3) Comme alkyl polyaminocarboxylates (APAC), on peut citer le cocoylpolyamino-carboxylate de sodium, commercialisé sous la dénomination AMPHOLAK 7 CX/C^{®} et AMPHOLAK 7 CX^{®} par la société Akzo Nobel, le stéaryl-polyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C^{®} par la société Akzo Nobel.
4) Comme alkylamphoacétates, on peut citer par exemple le N-cocoyl-N-carboxyméthoxyéthyl-N-carboxyméthyl-éthylènediamine N-di-sodique (nom INCI: disodium cocoamphodiacetate) comme le produit commercialisé sous la dénomination MIRANOL C2M CONCENTRE NP® par la société Rhodia, le N-cocoyl-N-hydroxyéthyl-N-carboxyméthyl-éthylènediamine N-sodique (nom INCI: sodium cocamphoacetate), le cocoamphohydroxypropyl sulfonate de sodium commercialisé sous la dénomination MIRANOL CSE par la société Rhodia.

Le second tensioactif moussant anionique peut être choisi notamment parmi les dérivés anioniques de protéines d'origine végétale ou de protéines de soie, les phosphates et alkylphosphates, les carboxylates, les sulfosuccinates, les dérivés des aminoacides, les alkyl sulfates, les alkyl éther sulfates, les sulfonates, les iséthionates, les taurates, les alkyl sulfoacétates, les polypeptides, les dérivés anioniques d'alkyl polyglucoside, et leurs mélanges.
1) Les dérivés anioniques de protéines d'origine végétale sont des hydrolysats de protéine à groupement hydrophobe, ledit groupement hydrophobe pouvant être naturellement présent dans la protéine ou être ajouté par réaction de la protéine et/ou de l'hydrolysat de protéine avec un composé hydrophobe. Les protéines sont d'origine végétale ou dérivées de soie, et le groupement hydrophobe peut être notamment une chaîne grasse, par exemple une chaîne alkyle comportant de 10 à 22 atomes de carbone. Comme dérivés anioniques de protéines d'origine végétale, on peut plus particulièrement citer les hydrolysats de protéines de pomme, de blé, de soja, d'avoine, comportant une chaîne alkyle ayant de 10 à 22 atomes de carbone et leurs sels. La chaîne alkyle peut être notamment une chaîne lauryle et le sel peut être un sel de sodium, de potassium et/ou d'ammonium.
   Ainsi, comme hydrolysats de protéine à groupement hydrophobe, on peut citer par exemple les sels des hydrolysats de protéine de soie modifiée par l'acide laurique, tels que le produit commercialisé sous la dénomination KAWA SILK par la société Kawaken ; les sels des hydrolysats de protéine de blé modifiée par l'acide laurique, tels que le sel de potassium commercialisé sous la dénomination Aminofoam W OR par la société Croda (nom INCI : Potassium lauroyl wheat aminoacids) et le sel de sodium commercialisé sous la dénomination PROTEOL LW 30 par la société Seppic (nom INCI : sodium lauroyl wheat aminoacids) ; les sels des hydrolysats de protéine d'avoine comportant une chaîne alkyle ayant de 10 à 22 atomes de carbone, et plus spécialement les sels des hydrolysats de protéine d'avoine modifiée par l'acide laurique, tels que le sel de sodium commercialisé sous la dénomination PROTEOL OAT (solution aqueuse à 30 %) par la société Seppic (nom INCI : Sodium lauroyl oat aminoacids) ; les sels des hydrolysats de protéine de pomme, comportant une chaîne alkyle ayant de 10 à 22 atomes de carbone, tels que le sel de sodium commercialisé sous la dénomination PROTEOL APL (solution hydroglycolique à 30%) par la société Seppic (nom INCI : Sodium Cocoyl Apple amino acids). On peut citer aussi le mélange de lauroyl-aminoacides (aspartique, glutamique, glycine, alanine) neutralisé au N-methylglycinate de sodium, commercialisé sous la dénomination PROTEOL SAV 50 S par la société Seppic (nom INCI : Sodium Cocoyl amino acids).
2) Comme phosphates et alkylphosphates, on peut citer par exemple les monoalkylphosphates et les dialkyl phosphates, tels que le mono-phosphate de lauryle commercialisé sous la dénomination MAP 20^{®} par la société Kao Chemicals, le sel de potassium de l'acide dodécyl-phosphorique, mélange de mono- et di-ester (diester majoritaire) commercialisé sous la dénomination CRAFOL AP-31^{®} par la société Cognis, le mélange de monoester et de di-ester d'acide octylphosphorique, commercialisé sous la dénomination CRAFOL AP-20^{®} par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER^{®} par la société Condea, le sel de potassium ou de triéthanolamine de monoalkyl (C₁₂-C₁₃) phosphate commercialisé sous les références ARLATONE MAP 230K-40^{®} et ARLATONE MAP 230T-60^{®} par la société Uniqema, le lauryl phosphate de potassium commercialisé sous la dénomination DERMALCARE MAP XC-99/09^{®} par la société Rhodia Chimie, et le cétylphosphate de potassium commercialisé sous la dénomination ARLATONE MAP 160K par la société Uniqema.
3) Comme carboxylates, on peut citer :
   - Les amido éthercarboxylates (AEC), comme le Lauryl amido ether carboxylate de sodium (3 OE), commercialisé sous la dénomination AKYPO FOAM 30^{®} par la société Kao Chemicals.
   - Les sels d'acides carboxyliques polyoxyéthylénés, comme le Lauryl ether carboxylate de sodium (C₁₂₋₁₄₋₁₆ 65/25/10) oxyéthyléné (6 OE) commercialisé sous la dénomination AKYPO SOFT 45 NV^{®} par la société Kao Chemicals, les acides gras d'origine huile d'olive polyoxyéthylénés et carboxyméthylés commercialisés sous la dénomination OLIVEM 400^{®} par la société BIOLOGIA E TECNOLOGIA, le tri-decyl ether carboxylate de sodium oxyéthyléné (6 OE) commercialisé sous la dénomination NIKKOL ECTD-6NEX^{®} par la société Nikkol.
   - Les sels d'acides gras ayant une chaîne alkyl en C₆ à C₂₂, neutralisés par une base organique ou minérale, qui constituent les savons. Le sel d'acide gras ou savon est obtenu à partir d'un acide gras et d'une base, l'acide gras comportant une chaîne alkyle linéaire ou ramifiée, saturée ou insaturée, ayant de 12 à 22 atomes de carbone et de préférence 12 à 20 atomes de carbone. Les bases (aussi appelées aussi agents de saponification) neutralisent totalement ou partiellement les acides gras. Les bases susceptibles d'être utilisées pour obtenir les sels peuvent être par exemple les bases minérales comme les hydroxydes de métaux alcalins (hydroxyde de sodium et potasse), les hydroxydes de métaux alcalino-terreux (de magnésium) ou l'hydroxyde d'ammonium, ou encore les bases organiques comme la triéthanolamine, la N-méthylglucamine, la lysine et l'arginine. L'acide gras peut être choisi en particulier parmi les acides gras en C₁₀ à C₂₄, et notamment en C₁₂-C₁₈, et en particulier l'acide laurique, l'acide myristique, l'acide stéarique, l'acide palmitique et leurs mélanges.
4) Comme dérivés des aminoacides, on peut citer notamment les sels alcalins d'aminoacides, tels que :
   - les sarcosinates, comme le Lauroyl sarcosinate de sodium commercialisé sous la dénomination SARKOSYL NL 97^{®} par la société Ciba ou commercialisé sous la dénomination ORAMIX L 30^{®} par la société Seppic, le myristoyl sarcosinate de sodium commercialisé sous la dénomination NIKKOL SARCOSINATE MN^{®} par la société Nikkol, le palmitoyl sarcosinate de sodium commercialisé sous la dénomination NIKKOL SARCOSINATE PN^{®} par la société Nikkol.
   - les alaninates, comme le N-lauroyl-N methyl amidopropionate de sodium commercialisé sous la dénomination SODIUM NIKKOL ALANINATE LN 30^{®} par la société Nikkol, ou commercialisé sous la dénomination ALANONE ALE^{®} par la société Kawaken, le N-lauroyl N-methyl alanine triéthanolamine commercialisé sous la dénomination ALANONE ALTA^{®} par la société Kawaken.
   - les glutamates, comme le mono-cocoyl glutamate de triéthanolamine commercialisé sous la dénomination ACYLGLUTAMATE CT-12^{®} par la société Ajinomoto, le lauroylglutamate de triéthanolamine commercialisé sous la dénomination ACYLGLUTAMATE LT-12^{®} par la société Ajinomoto.
   - les aspartates, comme le mélange de N-lauroyl aspartate de triethanolamine / N-myristoyl aspartate de triethanolamine commercialisé sous la dénomination ASPARACK^{®} par la société Mitsubishi.
   - les dérivés de glycine (glycinates), comme le N-cocoyl glycinate de sodium commercialisé sous les dénominations AMILITE GCS-12^{®} et AMILITE GCK 12 par la société Ajinomoto.
   - les citrates tels que le mono-ester citrique d'alcools de coco oxyéthylénés (9 moles), commercialisé sous la dénomination WITCONOL EC 1129 par la société Goldschmidt.
   - les galacturonates tels que le dodeécyl d-galactoside uronate de sodium commercialisé par la société Soliance.
5) Comme sulfosuccinates, on peut citer par exemple le mono-sulfosuccinate d'alcool laurylique (C₁₂/C₁₄ 70/30) oxyéthyléné (3 OE) commercialisé sous les dénominations SETACIN 103 SPECIAL^{®}, REWOPOL SB-FA 30 K 4^{®} par la société Witco, le sel di-sodique d'un hemi-sulfosuccinate des alcools C₁₂-C₁₄, commercialisé sous la dénomination SETACIN F SPECIAL PASTE^{®} par la société Zschimmer Schwarz, l'oléamidosulfosuccinate di-sodique oxyéthyléné (2 OE) commercialisé sous la dénomination STANDAPOL SH 135^{®} par la société Cognis, le mono-sulfosuccinate d'amide laurique oxyéthyléné (5 OE) commercialisé sous la dénomination LEBON A-5000^{®} par la société Sanyo, le sel di-sodique de mono-sulfosuccinate de lauryl citrate oxyéthyléné (10 OE) commercialisé sous la dénomination REWOPOL SB CS 50^{®} par la société Witco, le mono-sulfosuccinate de mono-éthanolamide ricinoléique commercialisé sous la dénomination REWODERM S 1333^{®} par la société Witco. On peut utiliser aussi les sulfosuccinates de polydimethylsiloxane tels que le disodium PEG-12 dimethicone sulfosuccinate commercialisé sous la dénomination MACKANATE-DC30 par la société Mac Intyre.
6) Comme alkyl sulfates, on peut citer par exemple le lauryl sulfate de triéthanolamine (nom INCI : TEA-lauryl sulfate) tel que le produit commercialisé par la société Huntsman sous la dénomination EMPICOL TL40 FL ou celui commercialisé par la société Cognis sur la dénomination TEXAPON T42, produits qui sont à 40 % en solution aqueuse. On peut aussi citer le lauryl sulfate d'ammonium (nom CFTA : Ammonium lauryl sulfate) tel que le produit commercialisé par la société Huntsman sous la dénomination EMPICOL AL 30FL qui est à 30 % en solution aqueuse.
7) Comme alkyl éther sulfates, on peut citer par exemple le lauryl éther sulfate de sodium (nom INCI : sodium laureth sulfate) comme celui commercialisé sous les dénominations TEXAPON N40 et TEXAPON AOS 225 UP par la société Cognis ou comme celui commercialisé sous la dénomination EMPICOL ESB 3/FL3 par la société Huntsman, le lauryl éther sulfate d'ammonium (nom INCI : ammonium laureth sulfate) comme celui commercialisé sous la dénomination STANDAPOL EA-2 par la société Cognis.
8) Comme sulfonates, on peut citer par exemple les alpha-oléfines sulfonates comme l'alpha-oléfine sulfonate de sodium (C₁₄-₁₆) commercialisé sous la dénomination BIO-TERGE AS-40^{®} par la société Stepan, commercialisé sous les dénominations WITCONATE AOS PROTEGE^{®} et SULFRAMINE AOS PH 12^{®} par la société Witco ou commercialisé sous la dénomination BIO-TERGE AS-40 CG^{®} par la société Stepan, l'oléfine sulfonate de sodium secondaire commercialisé sous la dénomination HOSTAPUR SAS 30^{®} par la société Clariant ; les alkyl aryl sulfonates linéaires comme le xylène sulfonate de sodium commercialisé sous les dénominations MANROSOL SXS30^{®}, MANROSOL SXS40^{®}, MANROSOL SXS93^{®} par la société Manro.
9) Comme iséthionates, on peut citer les acyliséthionates comme le cocoyl-iséthionate de sodium, tel que le produit commercialisé sous la dénomination JORDAPON CI P^{®} par la société Jordan.
10) Comme taurates, on peut citer le sel de sodium de méthyltaurate d'huile de palmiste commercialisé sous la dénomination HOSTAPON CT PATE^{®} par la société Clariant ; les N-acyl N-méthyltaurates comme le N-cocoyl N-methyltaurate de sodium commercialisé sous la dénomination HOSTAPON LT-SF^{®} par la société Clariant ou commercialisé sous la dénomination NIKKOL CMT-30-T^{®} par la société Nikkol, le palmitoyl methyltaurate de sodium commercialisé sous la dénomination NIKKOL PMT^{®} par la société Nikkol.
11) Les dérivés anioniques d'alkyl-polyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET^{®} par la société Cesalpinia, le sel di-sodique d'ester sulfosuccinique de cocoylpolyglucoside (1,4), commercialisé sous la dénomination ESSAI 512 MP^{®} par la société Seppic, le sel de sodium d'ester citrique de cocoyl polyglucoside (1,4) commercialisé sous la dénomination EUCAROL AGE-EC^{®} par la société Cesalpinia.

De préférence le premier tensioactif amphotère est choisi parmi N-alkylamidobétaines et leurs dérivés, en particulier la cocamidopropyl bétaine. De préférence le second tensioactif anionique choisi parmi les alkyl sulfates et leurs sels, par exemple le sodium lauryl sulfate.
Avantageusement le tensioactif amphotère et le tensioactif anionique sont présents dans la composition en un ratio tensioactif anionique / tensioactif amphotère allant de 0,7 à 1,5, par exemple de l'ordre de 1.

La composition selon l'invention peut également comprendre, outre le premier tensioactif amphotère et le second tensioactif anionique, un tensioactif moussant additionnel choisi par exemple parmi les tensioactifs non ioniques.

Les tensioactifs moussants non ioniques peuvent être choisis notamment parmi les alkyl polyglucosides (APG), les esters de glycérol oxyalkylénés, les esters de sucre oxyalkylénés, et leurs mélanges.
1) Comme alkylpolyglucosides, on utilise de préférence ceux contenant un groupe alkyle comportant de 6 à 30 atomes de carbone et de préférence de 8 à 16 atomes de carbone, et contenant un groupe glucoside comprenant de préférence 1, 2 à 3 unités de glucoside. Les alkylpolyglucosides peuvent être choisis par exemple parmi le decylglucoside (Alkyl-C9/C11-polyglucoside (1.4)) comme le produit commercialisé sous la dénomination Mydol 10^{®} par la société Kao Chemicals ou le produit commercialisé sous la dénomination Plantacare 2000 UP^{®} par la société Cognis ; le caprylyl/capryl glucoside comme le produit commercialisé sous la dénomination Plantacare KE 3711^{®} par la société Cognis ; le laurylglucoside comme le produit commercialisé sous la dénomination Plantacare 1200 UP^{®} par la société Cognis ; le cocoglucoside comme le produit commercialisé sous la dénomination Plantacare 818 UP^{®} par la société Cognis ; le caprylylglucoside comme le produit commercialisé sous la dénomination Plantacare 810 UP^{®} par la société Cognis ; et leurs mélanges.
2) Les esters de glycérol oxyalkylénés sont notamment les dérivés polyoxyéthylénés des esters de glycéryle et d'acide gras et de leurs dérivés hydrogénés. Ces esters de glycérol oxyalkylénés peuvent être choisis par exemple parmi les esters de glycéryle et d'acides gras hydrogénés et oxyéthylénés tel que le PEG-200 hydrogenated glyceryl palmate commercialisé sous la dénomination Rewoderm LI-S 80 par la société Goldschmidt ; les cocoates de glycéryle oxyéthylénés comme le PEG-7 glyceryl cocoate commercialisé sous la dénomination Tegosoft GC par la société Goldschmidt, et le PEG-30 glyceryl cocoate commercialisé sous la dénomination Rewoderm LI-63 par la société Goldschmidt ; et leurs mélanges.
3) Les esters de sucres oxyalkylénés sont notamment les éthers de polyéthylène glycol des esters d'acide gras et de sucre. Ces esters de sucre oxyalkylénés peuvent être choisis par exemple parmi les esters de glucose oxyéthylénés tels que le PEG-120 methyl glucose dioleate commercialisé sous la dénomination Glucamate DOE 120 par la société Amerchol.
   Selon un mode préféré de réalisation de l'invention, le tensioactif non ionique est un alkylpolyglucoside qui peut être choisi notamment parmi le decylglucoside, le caprylyl/capryl glucoside, le laurylglucoside, le cocoglucoside, le caprylylglucoside, et leurs mélanges.

Les tensioactifs moussants peuvent être présents en une teneur totale allant de 0,1 à 30% en poids par rapport au poids total de la composition, de préférence de 1 à 15% et mieux de 2 à 10% en poids.

### Agents gélifiants

La composition comprend moins de 1% d'agents gélifiants, de préférence moins de 0,5% d'agents et gélifiants et mieux, est exempte d'agents gélifiants.

Par agent gélifiant (aussi appelés agents épaississants), on entend un agent susceptible de modifier la viscosité de la composition. Ne sont pas considérés comme agents gélifiants les charges et particules exfoliantes citées plus loin.

A titre d'agents gélifiants, lipophiles ou hydrophiles, on peut citer :
- Les argiles organophiles qui sont des argiles modifiées par des composés chimiques rendant l'argile apte à gonfler dans les milieux huileux ; à titre d'exemples de tels produits, on peut citer les argiles de la famille des smectites telles que les montmorillonites, les hectorites, les bentonites, les beidellites, les saponites, ainsi que de la famille des vermiculites, de la stévensite, des chlorites.
   Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.
- Les silices pyrogénées qui peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-O-SIL HS-5®", "CAB-O-SIL EH-5®", "CAB-O-SIL LM-130®", "CAB-O-SIL MS-55®", "CAB-O-SIL M-5®" par la société Cabot.
- Les silices modifiées par des groupements hydrophobes tels que
   des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
   des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénomées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot,
- les homo- ou copolymères d'acides acrylique ou méthacrylique ou leurs sels et leurs esters et en particulier les produits vendus sous les dénominations « VERSICOL F » ou « VERSICOL K » par la société ALLIED COLLOID, « UTRAHOLD 8 » par la société CIBA-GEIGY, les acides polyacryliques de type SYNTHALEN K, et les sels, notamment de sodium, d'acide polyacryliques (répondant au nom INCI sodium acrylate copolymer) et plus particulièrement un polyacrylate de sodium réticulé (répondant au nom INCI sodium acrylate copolymer (and) caprylic/capric triglyceride) vendu sous la dénomination « LUVIGEL EM » par la société,
- les copolymères d'acide acrylique et d'acrylamide vendus sous la forme de leur sel de sodium sous les dénominations « RETEN » par la société HERCULES, le polyméthacrylate de sodium vendu sous la dénomination « DARVAN N°7 » par la société VANDERBILT, les sels de sodium d'acides polyhydroxycarboxyliques vendus sous la dénomination « HYDAGEN F » par la société HENKEL,
- les copolymères acide polyacryliques/acrylates d'alkyle de type PEMULEN,
- l'AMPS (Acide polyacrylamidométhyl propane sulfonique neutralisé partiellement à l'ammoniaque et hautement réticulé) commercialisé par la société CLARIANT,
- Les dérivés d'AMPS tels que
- les copolymères AMPS/acrylamide de type SEPIGEL ou SIMULGEL commercialisés par la société SEPPIC,
- les copolymères AMPS/méthacrylates d'alkyle polyoxyéthylénés (réticulé ou non) de type ARISTOFLEX HMS commercialisés par la société CLARIANT,
- et leurs mélanges.
- les protéines comme les protéines d'origine végétale telles que les protéines de blé, de soja ; les protéines d'origine animale tels que les kératines, par exemples les hydrolysats de kératine et les kératines sulfoniques ;
- les polymères de chitine ou de chitosane anioniques, cationiques, amphotères ou non-ioniques ;
- les polymères de cellulose et leurs dérivés tels que l'hydroxyéthylcellulose, l'hydroxypropylcellulose, la méthylcellulose, l'éthylhydroxyéthylcellulose, la carboxyméthylcellulose, ainsi que les dérivés quaternisés de la cellulose ;
- les polymères vinyliques, comme les polyvinylpyrrolidones, les copolymères de l'éther méthylvinylique et de l'anhydride malique, le copolymère de l'acétate de vinyle et de l'acide crotonique, les copolymères de vinylpyrrolidone et d'acétate de vinyle ; les copolymères de vinylpyrrolidone et de caprolactame ; l'alcool polyvinylique ;
- les polymères d'origine naturelle, éventuellement modifiés, tels que :

- les gommes arabiques, la gomme de guar, les dérivés du xanthane, la gomme de karaya ;
- les alginates et les carraghénanes ;
- les glycoaminoglycanes, l'acide hyaluronique et ses dérivés ;
- la résine shellac, la gomme de sandaraque, les dammars, les élémis, les copals ;
- l'acide désoxyribonucléïque ;
- les muccopolysaccharides tels que l'acide hyaluronique, les chondroïtines sulfate, et leurs mélanges.

En particulier, la composition est exempte de silices.

### Sels métalliques exothermiques

Les sels métalliques utilisés dans la présente invention sont des sels ayant des propriétés exothermiques, c'est-à-dire dégageant de la chaleur quand ils sont humidifiés. Ils sont choisis en particulier parmi les sels de métal alcalino-terreux, les sels de magnésium et leurs mélanges.

Comme sels de métal alcalino-terreux, on peut citer en particulier les sels de calcium et, plus spécialement, les halogénures de calcium tels que l'iodure, le chlorure et le bromure de calcium. Il s'agit de préférence du chlorure de calcium.

Comme sels de magnésium, on peut citer notamment le sulfate de magnésium.

On peut utiliser aussi un mélange de plusieurs sels.

Selon un mode préféré de réalisation de l'invention, le sel métallique exothermique est choisi parmi le sulfate de magnésium, le chlorure de calcium et leurs mélanges.

La quantité de sel(s) métallique(s) peut aller par exemple de 0,1 à 50 % en poids, de préférence de 5 à 40 % en poids et mieux de 10 à 30 % en poids par rapport au poids total de la composition.

### Polyols

Selon un mode préféré de réalisation de l'invention, la composition de l'invention contient en outre au moins un polyol car les polyols complètent l'action exothermique des sels.

A titre de polyols, on peut citer notamment les polyols ayant au moins 2 groupes hydroxyle et au moins 3 atomes de carbone, tels que la glycérine, la diglycérine, et les glycols tels que le propylène glycol, le dipropylène glycol, le butylène glycol, l'hexylène glycol, le polyéthylène glycol et les polyéthylène glycols de poids moléculaire inférieur à 600 comme le PEG-8 (ou Polyethylene 400), les sucres tels que le sorbitol, et leurs mélanges.

Comme polyols, on utilise de préférence la glycérine, le butylène glycol, le propylène glycol, le dipropylène glycol, le PEG-8 et leurs mélanges.

La quantité de polyol(s) dépend de la quantité de sels présents dans la composition et l'effet chauffant recherché. Elle peut aller par exemple de 0,5 à 80 % en poids, de préférence de 5 à 75 % en poids, mieux de 10 à 70 % en poids et encore mieux de 20 à 65 % en poids er encore mieux de 30 à 60 % en poids par rapport au poids total de la composition.

### Milieu anhydre

La composition selon l'invention comporte de préférence un milieu huileux comprenant au moins une huile. La quantité d'huile(s) peut aller par exemple de 5 à 80 % en poids, de préférence de 10 à 60 % en poids, mieux de 10 à 40 % en poids par rapport au poids total de la composition.

On entend par "huile" un corps gras liquide à la température ambiante (25°C).

Comme huiles utilisables dans la composition de l'invention, on peut citer par exemple :
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles d'amande douce, de tournesol, de maïs, de soja, de courge, de coriandre, de pépins de raisin, de sésame, de noisette, d'abricot (Prunus Armenica Oil), de macadamia, d'arara, de rUniqeman, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin ou 660084 PCL-LIQUID de la société SYMRISE (mélange d'éthyl-2 hexanoate de cétylstéaryle et de myristate d'isopropyle), l'isononanoate d'isononyle, le myristate d'isopropyle, le palmitate d'isopropyle, le palmitate d'éthyl-2-hexyle (ou palmitate d'octyle), le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de Parléam® ;
- les alcools gras ayant de 8 à 26 atomes de carbone, comme l'alcool cétylique, l'alcool stéarylique et leur mélange (alcool cétéarylique), l'octyldodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1^{®}" et "FLUTEC PC3^{®}" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluorométhoxybutane vendu sous la dénomination "MSX 4518^{®}" par la Société 3M et le nonafluoroéthoxyisobutane ; les dérivés de perfluoromorpholine, tels que la 4-trifluorométhyl perfluoromorpholine vendue sous la dénomination "PF 5052^{®}" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane et la cyclopentasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényl-diméthicones, les diphénylméthyl-diphényl trisiloxanes, les 2-phényléthyltriméthyl-siloxysilicates, et les polyméthyl-phénylsiloxanes ;
- leurs mélanges.

On entend par "huile hydrocarbonée" dans la liste des huiles citées ci-dessus, toute huile comportant majoritairement des atomes de carbone et d'hydrogène, et éventuellement des groupements ester, éther, fluoré, acide carboxylique et/ou alcool.

Quand la composition de l'invention est utilisée comme composition démaquillante, elle contient de préférence au moins une huile démaquillante, à laquelle on peut ajouter une ou plusieurs autres huiles démaquillantes ou non. Les huiles démaquillantes peuvent être choisies notamment parmi les hydrocarbures ramifiés d'origine minérale tels que le polyisobutène hydrogéné, les esters d'acide gras décrits ci-dessus, et leurs mélanges. Comme esters d'acide gras utilisables comme huiles démaquillantes, on peut citer plus particulièrement le palmitate d'éthyl hexyle, le stéarate d'éthyl hexyle, le myristate d'isopropyle, le palmitate d'isopropyle, le palmitate d'isobutyle, le caprate/caprylate de pentaérythritol, l'isononanoate de cétéaryle, l'isononanoate d'isodécyle, l'isononanoate d'isononyle, l'isononanoate d'isotridécyle, le caprate/caprylate d'éthyl-2 hexyle, et leurs mélanges.

En plus des huiles indiquées ci-dessus, la composition de l'invention peut contenir d'autres corps gras, tels que les acides gras comportant de 8 à 30 atomes de carbone, comme l'acide stéarique ; les résines de silicone telles que la trifluorométhyl-C1-4-alkyldimethicone et la trifluoropropyldimethicone ; les gommes de silicone (nom INCI : Dimethiconol) seules ou en mélange avec une huile de silicone, telles que le produit commercialisé par la société Dow Corning sous la dénomination Dow Corning 1501 Fluid, qui est un mélange de Dimethiconol et de Cyclopentasiloxane en proportion 14,7 / 85,3. (dimethiconol) ; les élastomères de silicone tels que ceux commercialisés sous les dénominations KSG par la société Shin-Etsu ; les cires, par exemple les cires minérales, les cires d'origine animale comme la cire d'abeille, les cires d'origine végétale, les huiles hydrogénées concrètes à 25°C, les esters gras et les glycérides concrets à 25°C, les cires synthétiques telles que la cire de polyméthylène, les cires de silicone ; et leurs mélanges.

### Additifs

La composition peut, en outre, contenir un ou plusieurs autres additifs, notamment lipophiles, classiquement utilisés dans les compositions cosmétiques.

On peut citer notamment les composés exothermiques autres que les sels et les polyols, comme par exemple les zéolithes qui sont des silicoaluminates. A titre de zéolites, on peut citer notamment les zéolites activées, et par exemple les zéolites A, les zéolites X comme celles commercialisées par les sociétés Fluka et Union Carbide, les zéolites MAP telles que décrites dans le document EP-A-384070, les zéolites A activées telles que décrites dans le document EP-A-187912 et notamment celles commercialisées sous le nom de ADVERA 401 N ET ADVERA 402N par la société PQ Corporation.

On peut aussi citer comme additifs, les parfums ; les conservateurs ; les antioxydants ; les séquestrants ; les charges ; les colorants, les actifs cosmétiques ou dermatologiques, ou leurs mélanges. Ces adjuvants sont utilisés dans les proportions habituelles des compositions nettoyantes et/ou de soin, et par exemple de 0,01 à 10 % du poids total de la composition. Ces adjuvants doivent être de nature et utilisés en quantité telles qu'ils ne perturbent pas les propriétés recherchées pour la composition de l'invention.

Selon un mode particulier de réalisation de l'invention, la composition selon l'invention contient comme charges des particules exfoliantes qui vont permettre le gommage de la peau. Comme particules exfoliantes, on peut utiliser des particules exfoliantes ou gommantes d'origines minérale, végétale ou organique. Ainsi, on peut utiliser par exemple les billes ou la poudre de polyéthylène, comme celles commercialisées sous la dénomination Microthene MN 727 ou Microthene MN 710-20 par la société Equistar ou comme la poudre commercialisée sous la dénomination Gotalene 120 Incolore 2 par la société Dupont ; les particules de Nylon comme celles commercialisées par la société Arkema sous la dénomination Orgasol 2002 EXD NAT COS ; les fibres comme les fibres de polyamide, comme celles commercialisées par la société Utexbel sous la dénomination PULPE POLYAMIDE 12185 TAILLE 0,3 MM ; la poudre de polychlorure de vinyle ; la pierre ponce (nom INCI : pumice) comme le ponce 3/B d'Eyraud ; les coques de noyaux de fruits broyées comme les broyats de noyaux d'abricots ou de coques de noix ; la sciure de bois ; les billes de verre ; l'alumine (oxyde d'aluminium) (nom INCI : Alumina) comme le produit commercialisé sous la dénomination Dermagrain 900 par la société Marketech International, ;les cristaux de sucre ; des billes qui fondent lors de l'application sur la peau, telles que par exemple, les sphères à base de mannitol et cellulose commercialisées sous les dénominations Unisphères par la société Induchem, les capsules à base d'agar commercialisées sous les dénominations Primasponge par la société Cognis, et les sphères à base d'esters de jojoba commercialisées sous les dénominations Florasphères par la société Floratech ; et leurs mélanges.

La composition selon l'invention peut aussi contenir d'autres charges telles que, par exemple, le talc, l'amidon modifié ou non, et notamment les amidons estérifiés par l'anhydride octénylsuccinique et plus particulièrement le "Aluminium Starch octenyl succinate" tel que le produit commercialisé par la société National Starch sous le nom de Dry-Flo,.

Ces charges (exfoliantes et autres) peuvent être présentes en une quantité allant par exemple de 0,5 à 20 % en poids, de préférence de 1 à 15 % en poids, mieux de 1 à 10 % et encore mieux de 2 à 5 % en poids par rapport au poids total de la composition.

Selon un autres mode particulier de réalisation, la composition selon l'invention est exempte de charges ou particules exfoliantes telles que décrites ci-dessus.

Comme actifs, on peut citer tout actif de soin ou de nettoyage habituellement utilisé dans le domaine cosmétique, et en particulier des antibactériens comme l'octopirox et le triclosan, des agents kératolytiques comme l'acide salicylique, l'acide lactique ou l'acide glycolique, des huiles essentielles, des vitamines comme la vitamine C (acide ascorbique, la vitamine A (rétinol), la vitamine PP (niacinamide), la vitamine B3 (panthénol) et leurs dérivés.

La composition de l'invention est particulièrement adaptée pour le nettoyage et/ou le démaquillage de la peau et/ou des muqueuses et plus particulièrement pour le nettoyage et/ou le démaquillage et/ou le gommage de la peau.
L'invention a encore pour objet l'utilisation cosmétique de la composition telle que définie ci-dessus, pour le nettoyage et/ou le démaquillage et/ou le gommage de la peau.

La composition de l'invention se présente généralement sous forme d'un gel. Lors de l'utilisation, on applique la composition sur la peau (notamment la peau du visage), de préférence en couche épaisse, puis on humidifie les mains et on masse le visage avec les mains mouillées. On peut éventuellement réhumidifier les mains et masser à nouveau le visage. On termine en rinçant le visage. Un autre procédé consiste à humidifier d'abord la peau, puis à appliquer la composition sur la peau humide et à masser le visage, après avoir ou non humidifié les mains.

Ainsi, l'invention a encore pour objet un procédé cosmétique de démaquillage et/ou de nettoyage et/ou de gommage de la peau, consistant à humidifier la peau, à appliquer sur la peau, la composition telle que définie ci-dessus, à masser et à rincer la peau..

L'invention a aussi pour objet un procédé cosmétique de démaquillage et/ou de nettoyage de la peau, consistant à appliquer sur la peau, la composition telle que définie ci-dessus, à mouiller les mains, à masser le visage avec les mains mouillées, et à rincer la peau.

La composition de l'invention peut être pour adaptée le rasage de la peau par exemple du visage ou des jambes.

C'est pourquoi selon un aspect, l'invention a encore pour objet un procédé de rasage consistant à appliquer sur la surface de la peau à raser une composition telle que définie dans l'une quelconque des revendications précédentes puis à raser les poils au moyen d'un rasoir.
L'exemple ci-après est donné à titre illustratif et non limitatif en vue de mieux faire ressortir les caractéristiques de l'invention. Les quantités y sont données en % en poids.

### Exemple 1 : Composition chauffante moussante

- PEG-8 qsp 100 %
- Glycérine 20 %
- Mélange sulfate de magnésium et eau (98/2) 20 %
- Acide citrique 0,5 %
- Cocoamidopropylbetaïne 5 %
- Sodium lauryl sulfate (EMPICOL TL40 FL de Huntsman) 5 %

## Revendications

1. Composition cosmétique comprenant dans un milieu anhydre, au moins un sel métallique exothermique, au moins au moins un premier tensioactif moussant amphotère et au moins un second tensioactif moussant anionique présents en un ratio tensioactif anionique / tensioactif amphotère allant de 0,5 à 2, ladite composition comprenant moins de 1 % d'agent gélifiant.

2. Composition selon la revendication 1, **caractérisée en ce que** le sel métallique exothermique est choisi parmi les sels de métal alcalino-terreux, les sels de magnésium et leurs mélanges.

3. Composition selon l'une quelconque des revendications précédentes, **caractérisée en ce que** le sel métallique exothermique est choisi parmi le sulfate de magnésium, le chlorure de calcium et leurs mélanges.

4. Composition selon l'une quelconque des revendications précédentes, **caractérisée en ce que** la quantité de sel(s) métallique(s) exothermique(s) va de 0,1 à 50 % en poids par rapport au poids total de la composition.

5. Composition selon l'une des revendications précédentes, **caractérisée en ce que** le premier tensioactif amphotère est choisi parmi les N-alkylamidobétaines et leurs dérivés.

6. Composition selon l'une des revendications précédentes, **caractérisée en ce que** le premier tensioactif amphotère est la cocamidopropyl bétaine.

7. Composition selon l'une des revendications précédentes, **caractérisée en ce que** le second tensioactif anionique est choisi parmi les alkyl sulfates et leurs sels.

8. Composition selon l'une des revendications précédentes, **caractérisée en ce que** le second tensioactif anionique est le sodium lauryl sulfate.

9. Composition selon une des revendications précédentes, **caractérisée en ce que** le tensioactif amphotère et le tensioactif anionique sont présents dans la composition en un ratio tensioactif anionique / tensioactif amphotère allant de 0,7 à 1,5.

10. Composition selon l'une des revendications précédentes, **caractérisée en ce que** les tensioactifs moussants sont présents en une teneur totale allant de 0,1 à 30% en poids par rapport au poids total de la composition, de préférence de 1 à 15% et mieux de 2 à 10% en poids.

11. Procédé cosmétique de démaquillage et/ou de nettoyage et/ou de gommage de la peau, consistant à humidifier la peau, à appliquer sur la peau la composition selon l'une quelconque des revendications 1 à 10, à masser et à rincer la peau.

12. Procédé de rasage consistant à appliquer sur la surface de la peau à raser une composition selon l'une quelconque des revendications 1 à 10 puis à raser les poils au moyen d'un rasoir.
